**Europäisches Patentamt**

**European Patent Office**

**Office européen des brevets**

(19)

(11) Publication number : **0 452 071 B1**

# (12) EUROPEAN PATENT SPECIFICATION

(45) Date of publication of patent specification :
26.07.95 Bulletin 95/30

(51) Int. Cl.⁶ : **G01N 27/20, G01N 17/04**

(21) Application number : **91303085.4**

(22) Date of filing : **09.04.91**

(54) Method and device for diagnosis of paint film deterioration.

(30) Priority : **09.04.90 JP 93637/90**
**09.01.91 JP 12865/91**

(43) Date of publication of application :
**16.10.91 Bulletin 91/42**

(45) Publication of the grant of the patent :
**26.07.95 Bulletin 95/30**

(84) Designated Contracting States :
**DE FR GB**

(56) References cited :
**EP-A- 0 245 116**
**GB-A- 2 195 771**
**US-A- 4 806 849**
**PATENT ABSTRACTS OF JAPAN vol. 11, no. 311 (P-625), 12 October 1987 ; & JP-A-62 102 148**
**IBM TECHNICAL DISCLOSURE BULLETIN, vol. 29, no. 12, May 1987, New York, US, pp. 5565-5566 ; Anonymous: "Defect detection in conformal organic coatings of metal foils by AC impedance"**

(56) References cited :
**JOURNAL OF PHYSICS E. SCIENTIFIC IN-STRUMENTS, vol. 22, no. 5, May 1989, ISHING, BRISTOL, GB, pp. 293-296 ; W. LORTZ et al. : "High-temperature cell for automated low-frequency impedance measurements of sol-ids"**
**PATENT ABSTRACTS OF JAPAN vol. 10, no. 322 (P-511)(2378) 31 October 1986 ; & JP-A-61 128 151**

(73) Proprietor : **KABUSHIKI KAISHA TOSHIBA**
**72, Horikawa-cho,**
**Saiwai-ku**
**Kawasaki-shi, Kanagawa-ken 210, Tokyo (JP)**

(72) Inventor : **Kondou, Takeshi, Mie Works, K.K. Toshiba**
**2121, Oaza Nao,**
**Asahi-cho**
**Mie-gun, Mie-ken (JP)**
Inventor : **Yamamoto, Sumio, Keihin Product Operations**
**K.K. Toshiba,**
**2-4, Suehiro-cho,**
**Tsurumi-ku**
**Yokohama-shi, Kanagawa-ken (JP)**

(74) Representative : **Freed, Arthur Woolf et al**
**MARKS & CLERK,**
**57-60 Lincoln's Inn Fields**
**London WC2A 3LS (GB)**

## Description

This invention relates generally to a method and device for determining the degree of aged deterioration of a paint film formed on the surface of a metal member of an equipment, and more particularly to such a paint film deterioration diagnosing method and device wherein the degree of deterioration of the paint film is determined from an impedance of the paint film.

A paint film is usually formed on the outer surface of an equipment installed outdoors, for example, transformers or switchboards for the purposes of corrosion prevention, appearance improvement and the like. It is known in the art that the paint film formed on the outer surface of the equipment is deteriorated with time at different speeds depending upon environment of the equipment. When the paint film is left deteriorated, corrosion of a base metal covered by the paint film finally causes flaking of the paint film and occurrence of rust. The equipment needs to be repainted at an appropriate time for maintenance thereof so that a new paint film is formed on the outer surface of the equipment. When repainting is carried out too early, it results in wasting the cost therefor. When repainting is carried out too late, an economic burden is increased since repair of the corroded base metal takes much time. Thus, when an opportunity for repainting is missed, a bad influence is exerted on the maintenance of the equipment. Conventionally, in the above-mentioned equipments, the degree of deterioration of the paint film is periodically diagnosed so that an appropriate opportunity for repainting is determined.

It is desirable that the result of the above-mentioned diagnosis should have as much objectivity as possible or an appropriate determination should always be made no matter who carries out the diagnosis. In consideration of this point, the prior art has provided the following method of diagnosing the deterioration of the paint film. A large number of sample photographs are previously prepared which show different stages of deterioration with respect to various conditions of deterioration such as peeling, cracking, blistering, rusting and the like. The paint film to be actually diagnosed is compared with the sample photographs by a workman engaged in the diagnosis. The workman understands what stages of the sample photographs the condition of the paint film to be diagnosed corresponds to with respect to various conditions of deterioration and further, assigns predetermined scores with respect to various conditions and degrees of deterioration. The scores are sequentially added. When the value obtained from addition of scores reaches a predetermined value, it is determined by the workman that repainting or repair and repainting should be carried out.

The prior art has provided another method of diagnosing the paint film deterioration, which method is somewhat in advance of the above-described one. This method makes use of an impedance of the paint film varied depending upon the degree of deterioration. This method is disclosed in Japanese Laid-open (kokai) Patent Application No. 62-102148 (1987) filed by the assignee of the present application, for example. An actually employed diagnosis procedure in accordance with this method is summarized as follows. An impedance measurement device is brought into a place where the equipment as an object to be diagnosed is installed. A probe is applied to the surface of the paint film and voltages at various frequencies are applied across the probe and a base metal on which the paint film is formed. The paint film impedance values measured based on the currents flowing as the result of voltage application are read by the workman to be written down. The resistive and capacitive impedance values written down with respect to various frequencies are compared with a previously prepared list for determining what columns in the list the impedance values correspond to, thereby determining the degree of the paint film deterioration. The frequency for measuring the paint film impedance is usually 200 Hz, 500 Hz or 1 KHz.

In the former method, however, the determination to which of the sample photographs the actual degree of the paint film deterioration corresponds tends to be influenced by the subjectivity of the workman engaged in the diagnosis. Thus, this method cannot warrant that every workman can always carry out an appropriate diagnosis. Accordingly, the result of the diagnosis is not likely to be highly esteemed and therefore, repainting or repair of the equipment with painting is not carried out at an appropriate time. Furthermore, this method necessitates for the workman to compare the actual paint film with a number of sample photographs and to add the scores. These works are troublesome and miscalculations can occur in adding the scores.

Patent Abstracts of Japan, vol. 11, no. 311 (P-625) (see above JP-A-62 102148) disclose a method for diagnosing the deterioration of a coating film. In this method the substance whose deterioration is to be measured and a current measuring instrument are connected in series to an AC power supply with the result that a synthesized waveform is outputted. Voltage applied to the substance as well as the current flowing through the substance are detected. The impedance of the substance is calculated from the voltage and current and the output impedance value is used as a measure of deterioration.

On the other hand, in the latter method in which the impedance of the paint film is measured, there is a possibility that readings of measured values may be influenced by the operator's subjectivity. Additionally, it is troublesome to compare the measured values with criterion for evaluation.

2

EP 0 452 071 B1

Apparatus for measuring the resistivity of fibre reinforced plastics and coatings for calculating the effective life of the coating by means of non-linear regression analysis and extrapolation is disclosed in EP-A-0245116.

Therefore, an object of the present invention is to provide an improved method and device for diagnosis of paint film deterioration wherein the diagnosis of the paint film deterioration can be prevented from being influenced by the subjectivity of a person engaged in the diagnosis so that an appropriate result of diagnosis can be obtained and the diagnosing works can be carried out with ease and accuracy.

In one aspect, the present invention provides a method of diagnosing the degree of deterioration of a paint film based on an impedance comprising the steps of measuring an impedance by applying a probe to the surface of the paint film, applying voltage across the probe and a base metal on which the paint film is formed, the voltage having a frequency range of 0.01 to 1 Hz, and measuring the resulting current based upon the applied voltage across the probe, characterized by the steps of converting an analog signal indicative of the measured current to a corresponding digital impedance signal, providing a microcomputer with an operational program representative of a functional expression corresponding to an orthogonal graph having an axis representative of logarithmic values of impedance and an axis representative of an elapsed service time period in the year, the operational program including means for converting an impedance value to a logarithmic value, inputting to the microcomputer digital data of an initial impedance of the paint film measured at the time of formation of the paint film, the digital impedance signal, digital data of an elapsed time period from the time of formation of the paint film until the measurement of impedance of the paint film, and a predetermined life-end-point impedance of the paint film, performing an operation for obtaining an equation representative of a straight line passing through the initial impedance and the measured impedance on said orthogonal graph, performing an operation for obtaining in the microcomputer a life-end-point of the paint film by substituting the predetermined life-end-point impedance into the equation representative of the straight line passing through the initial impedance and the measured impedance, obtaining, in the microcomputer, a remaining life-duration of the paint film from the time of measurement of the impedance of the paint film to the life end point, and displaying the obtained remaining life-duration of the paint film on a display.

In accordance with the above-described method, when a voltage is applied by the voltage applying means across the base metal and the probe, a current is caused to flow into the probe through the paint film. The current flowing into the probe is measured by the current measuring means. An analog signal obtained by the current measuring means is converted to a corresponding digital signal by the analog-to-digital conversion means and the digital signal is inputted to a computer. It is known that the magnitude and phase of a current flowing into a paint film, that is, an impedance of the paint film has a correlation with the degree of deterioration of the paint film. The degree of deterioration of the paint film is determined by the computer from the digital signal outputted by the analog-to-digital conversion means in accordance with an analysis procedure which is previously determined in consideration of conditions of the applied voltage, frequency and the like and the above-mentioned correlation. The result of determination is outputted by the output means. What is necessary for the workman to do is to apply the probe on the surface of the paint film to be diagnosed and to operate the computer so that the degree of deterioration of the paint film is determined. Consequently, an objective determination result can be obtained.

In the above-described method, the degree of deterioration may be determined by the computer from the initial impedance value of the paint film, the elapsed time period after formation of the paint film, the measured impedance value and the predetermined life-end-point impedance of the paint film.

Accordingly, the degree of deterioration of the paint film can be accurately determined when the initial impedance and the life-end-point impedance with respect to the kinds of paints of the paint film is previously obtained from, for example, experiments in consideration of these relations found by the inventors. Consequently, an appropriate time for repainting, that is, the remaining life-duration of the paint film can be accurately determined.

In another aspect, the present invention provides an apparatus for diagnosing the degree of deterioration of a paint film based on an impedance comprising means for measuring an impedance by applying a probe to the surface of the paint film, applying voltage across the probe and a base metal on which the paint film is formed, the voltage having a frequency range of 0.01 to 1 Hz, and measuring the resulting current based upon the applied voltage across the probe, characterized by means for converting an analog signal indicative of the measured current to a corresponding digital impedance signal, means for providing a microcomputer with an operational program representative of a functional expression corresponding to an orthogonal graph having an axis representative of logarithmic values of impedance and an axis representative of an elapsed service time period in the year, the operational program including means for converting an impedance value to a logarithmic value, means for inputting to the microcomputer digital data of an initial impedance of the paint film measured at the time of formation of the paint film, the digital impedance signal, digital data of an elapsed time period from the time of formation of the paint film until the measurement of impedance of the paint film,

3

and a predetermined life-end-point impedance of the paint film, means for performing an operation for obtaining an equation representative of a straight line passing through the initial impedance and the measured impedance on said orthogonal graph, means for performing an operation for obtaining in the microcomputer a life-end-point of the paint film by substituting the predetermined life-end-point impedance into the equation representative of the straight line passing through the initial impedance and the measured impedance, means for obtaining, in the microcomputer, a remaining life-duration of the paint film from the time of measurement of the impedance of the paint film to the life end point, and means for displaying the obtained remaining life-duration of the paint film on a display.

In accordance with the above-described method and apparatus, even when the electrical equipments to be diagnosed, for example, transformers, are located far away, the workman goes round to the locations of the equipments with a current measuring device carried in order to measure the current flowing into the probe. Only the storage medium is brought into central maintenance facilities, where the storage medium is attached to the determination device. Thus, the determination of deterioration of the paint film can be carried out collectively with respect to a large number of electrical equipments. Consequently, the diagnosis of the paint film deterioration can be controlled with ease.

The invention will be described, merely by way of example, with reference to the accompanying drawings, in which:

FIG. 1 is a block diagram of a paint film deterioration diagnosis device of an embodiment in accordance with the present invention;

FIG. 2 is a graph showing an assumed life line of the paint film;

FIG. 3 illustrates an example of waveform of a voltage applied to the paint film;

FIG. 4 shows an example of the diagnosis result on display;

FIG. 5 is a flowchart explaining a procedure of diagnosis in accordance with a diagnosis program;

FIG. 6 is a graph showing another example of the assumed life line of the paint film;

FIG. 7 is a graph showing a relation between the paint film impedance and the temperature;

FIG. 8 is a graph of measured data showing the relation between the paint film impedance and the temperature; and

FIG. 9 is a view similar to FIG. 1 illustrating a modified form of the paint film deterioration diagnosis device.

An embodiment of the present invention will be described with reference to the accompanying drawings. Reference numeral 1 designates a so-called lap-top type personal computer. A casing (not shown) thereof encloses a central processing unit (CPU) 2, a read only memory (ROM) 3, a random access memory (RAM) 4, a floppy disc drive (FDD) 5 and the like. The personal computer 1 includes a liquid crystal display 6 and a keyboard 7. An interface (I/F) board 8 with analog-to-digital and digital-to-analog conversion functions is inserted in an expansion slot of the personal computer 1.

A probe 10 is applied to a paint film 9 to be diagnosed with respect to deterioration thereof. Various types of such probes are well known. See Japanese Laid-open (kokai) Patent Application No. 61-108954, for example. Basically, the probe 10 comprises a container containing spongy electrodes impregnated with a conductive gel and a permanent magnet provided along the peripheral edge of an opening of the container. The spongy electrodes of the probe 10 are closely adhered to the surface of the paint film 9 by an attractive force incurred between the permanent magnet and a base metal 11 on which the paint film 9 is formed. A lead wire extended from the probe 10 and another lead wire connected to the base metal 11 are connected to a preamplifier 12 so that a voltage signal in accordance with a current flowing into the probe 12 through the paint film 9 when voltage is applied across the probe 10 and the base metal 11 is supplied to the I/F board 8 of the personal computer 1. The preamplifier 12 thus serves as current measuring means for measuring a current flowing into the probe 10 across the paint film 9. This current measurement corresponds to measuring an impedance of the paint film 9. The preamplifier 12 is designed to switch its measurement ranges depending upon degree of deterioration of the paint film 9. In the embodiment, the preamplifier 12 covers a measurable current range between 0.01 $\mu$A and 10 $\mu$A, the range corresponding to an impedance range between 0.1 M$\Omega$ and 100 M$\Omega$.

A procedure for diagnosing the paint film 9 with the deterioration diagnosis device thus arranged will now be described with functions of the personal computer 1.

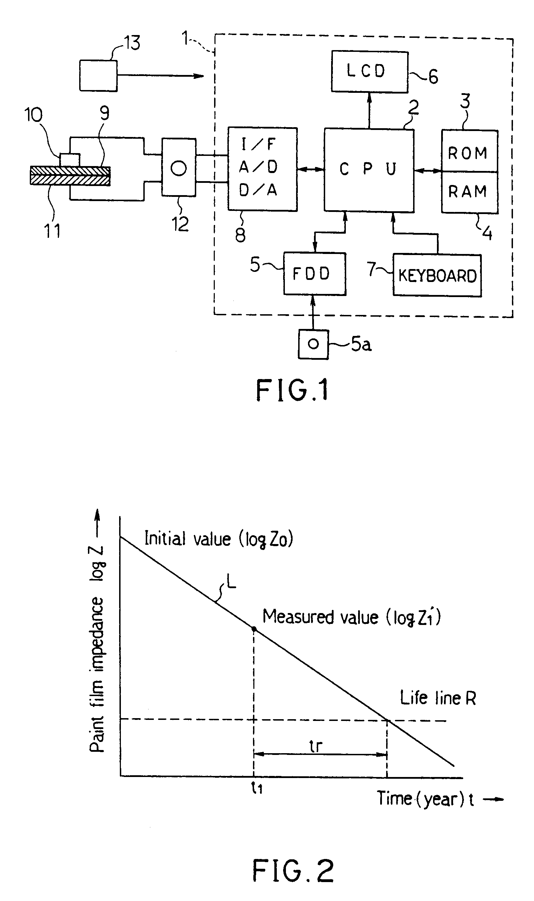

First, the personal computer 1, the preamplifier 12 and the probe 10 are brought into a place where the diagnosis of deterioration is carried out. The probe 10 is applied to the surface of the paint film 9 and then connected to the preamplifier 12 which is further connected to the personal computer 1, as shown in FIG. 1. A floppy disc 5a storing data of a measurement program is inserted into FDD 5 so that the personal computer 1 inputs the data from the floppy disc 5a and executes the measurement program. As the result of execution of the measurement program, a voltage is applied across the probe 10 and the base metal 11 and a current flowing into the paint film 9 at the time of the voltage application is detected by the preamplifier 12, which then, supplies the I/F board 8 of the personal computer 1 with a voltage signal indicative of the detected current.

The analog voltage signal supplied to the I/F board 8 is converted to a corresponding digital signal by the A/D conversion function thereof. The digital signal produced from the I/F board 8, that is, data of the measured impedance is written into the floppy disc 5a in FDD 5 under the control of CPU 2. Such a paint film impedance measurement as described above is carried out with respect to a plurality of measurement portions on the paint film surface. Measurement data of the temperature T of the paint film 9 or the equipment measured by temperature measuring means 13 at the time of measurement of the paint film impedance, the thickness of the paint film 9, the area of the probe 10 in contact with the paint film surface and the like is inputted at the keyboard 7 of the personal computer 1 along with measurement of the paint film impedance, which data being registered in the floppy disc 5a.

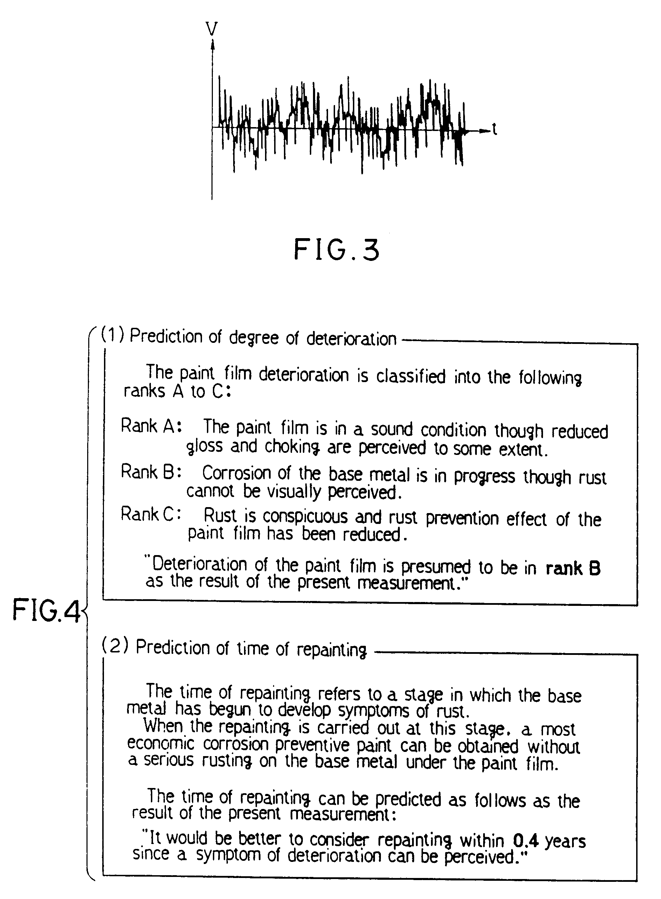

A waveform of the voltage applied across the probe 10 and the base metal 11 is generated and composed by the D/A conversion function of the I/F board 8 based on digital data previously stored in the floppy disc 5a. The I/F board 8 thus serves as voltage applying means for applying a voltage with a predetermined waveform across the probe 10 and the base metal 11 covered by the paint film 9. In the embodiment, the frequency of the applied voltage ranges from 0.01 Hz to 1 Hz and power spectrum P is between $P = 1/\sqrt{f}$ and $P = 1/f^2$. FIG. 3 shows an example of the voltage waveform composed so that the power spectrum P becomes $P = 1/f$. The phase of each frequency component composing the voltage waveform is randomized so that a peak voltage is suppressed to the utmost. The inventors have found that the logarithm log Z of the paint film impedance has a linearity relative to time t when the measurement frequency ranges from 0.01 Hz to 1 Hz, as is shown in FIG. 2. An analysis of degree of the paint film deterioration can be carried out with ease and accuracy when log Z has the linearity relative to time t, as will be described later. The range of the power spectrum P is determined to be between $P = 1/\sqrt{f}$ to $P = 1/f^2$ for the following reason. That is, it is desirable that an amount of a low-frequency component should be increased with an amount of a high-frequency component decreased from the point of view that the magnitude of the measured current is increased but it is undesirable that the amount of the high-frequency component should be excessively decreased from the point of view that error of measurement is reduced, as described in detail in above Japanese Laid-open (kokai) Patent Application No. 61-102148 (1987) applied for patent by the assignee of the present application. Consequently, the current or the paint film impedance can be measured with high accuracy when the voltage waveform is determined such that the power spectrum P as described above is obtained.

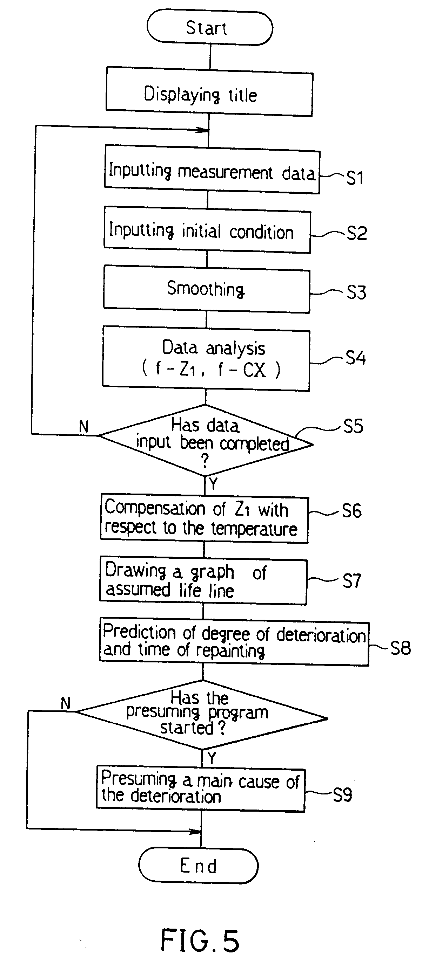

Upon completion of the measurement of the current or paint film impedance with the probe 10 as described above, the personal computer 1 inputs data of a diagnosis program from the floppy disc 5a and carries out the diagnosis program. A procedure of the diagnosis program will be described with reference to a flowchart of FIG. 5.

Step S1: input of data obtained from measurement:

Measurement data is read out from the floppy disc 5a and the data of the temperature T of the paint film 9 at the time of the impedance measurement, the thickness thereof, the area of the probe 10 in contact with the paint film surface and the like is displayed on the display 6 for confirmation. Subsequently, upon an operation of confirmation, the display 6 is in the initial condition input mode.

Step S2: initial condition input:

Codes representative of the kind of paint of the paint film, the number of elapsing years after formation of the paint film, state of appearance, environment of the equipment on which the paint film is formed, an initial impedance $Z_0$- and the like are inputted at the keyboard 7 of the personal computer 1. Three selective elements, that is, the phthalic acid system, the epoxy-urethane system and the others, are provided as the kinds of paints. One of them is selected regarding the kind of paint of the paint film.

Step S3: smoothing:

The obtained data of measured impedance is smoothed. Since data of the measured impedances is not always varied uniformly relative to each frequency, the impedance data is smoothed for improvement of the measurement accuracy.

Step S4: Data analysis:

The value of paint film impedance $Z_1$ at the frequency of 0.1 Hz is obtained based on the smoothed impedance data. For confirmation of suitability of measurement conditions, a graph representing the paint film

impedance $Z_1$ and the capacity component Cx is constructed and displayed.

In this case, the paint film impedance Z is considered to be equivalent to a circuit comprising a parallel circuit of a resistance and a capacitor. An amount of resistance component is infinitely increased when the paint film 9 is in the normal condition. Since the amount of resistance component is decreased with deterioration of the paint film 9, the impedance Z is reduced Particularly in the low frequency range. Accordingly, the condition of deterioration of the paint film 9 can be roughly comprehended from the above-mentioned graph.

The condition of corrosion of the base metal 11 can also be determined from the graph of the relation between the frequency f and the capacity component Cx. More specifically, the capacity component Cx takes a fixed value relative to the frequency f when the paint film is in the normal condition. However, the capacity of a so-called electrical double layer is increased when the base metal corrodes.

Step S5: repetition of data analysis and determination of abnormal data:

Since the measurement of the paint film impedance $Z_1$ is carried out on a plurality of measurement portions on the surface of an equipment, the above-described data analysis is repeated by the number of the measurement portions. The mean value and dispersion of the values of the paint film impedance $Z_1$ at the frequency of 0.1 Hz at the respective measurement portions are calculated. Abnormal data is eliminated by the t-distribution method as resulting from failures in measurement.

Step S6: compensation of the paint film impedance $Z_1$ with respect to the temperature:

The value of the detected impedance $Z_1$ is varied to a large extent depending upon the temperature of the paint film at the time of measurement. Therefore, the mean value of the impedance $Z_1$ is converted to the paint film impedance $Z_1'$ at a reference temperature of 25°C, for example.

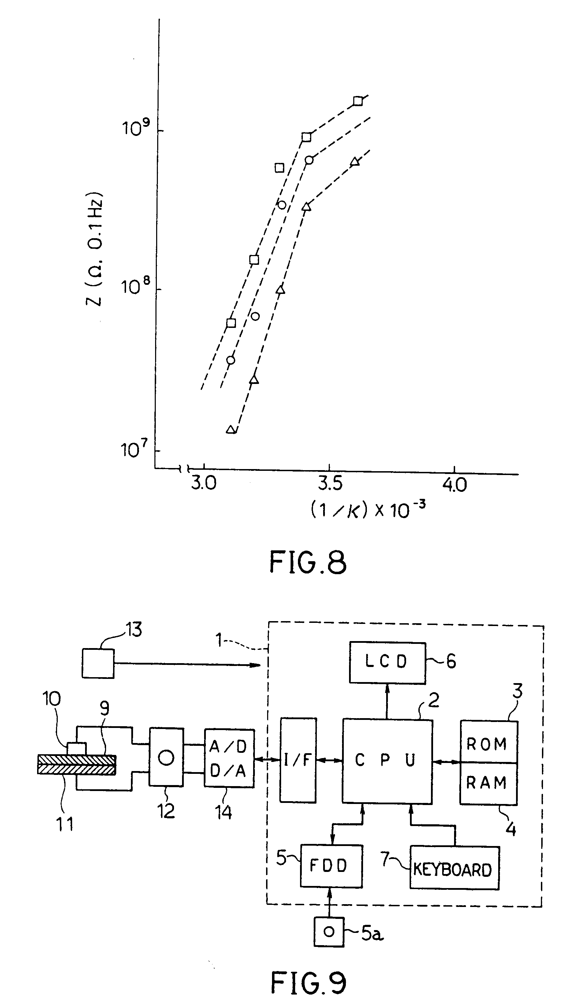

FIG. 8 shows measured data representing the relationship between the inverse numbers of the temperatures T (°K) of three kinds of paint films 9 and the logarithm log Z of the impedance Z. As understood from FIG. 8, the value of log Z is varied depending upon the temperature T and the variation of log Z is represented as two continuous straight lines having inclinations different from each other with the value of about 25°C (about 298°K) as the transition point. Accordingly, the reference temperature is established as 25°C and the value of impedance $Z_1$ obtained at the temperature T is compensated for as the impedance value at the reference value, 25°C.

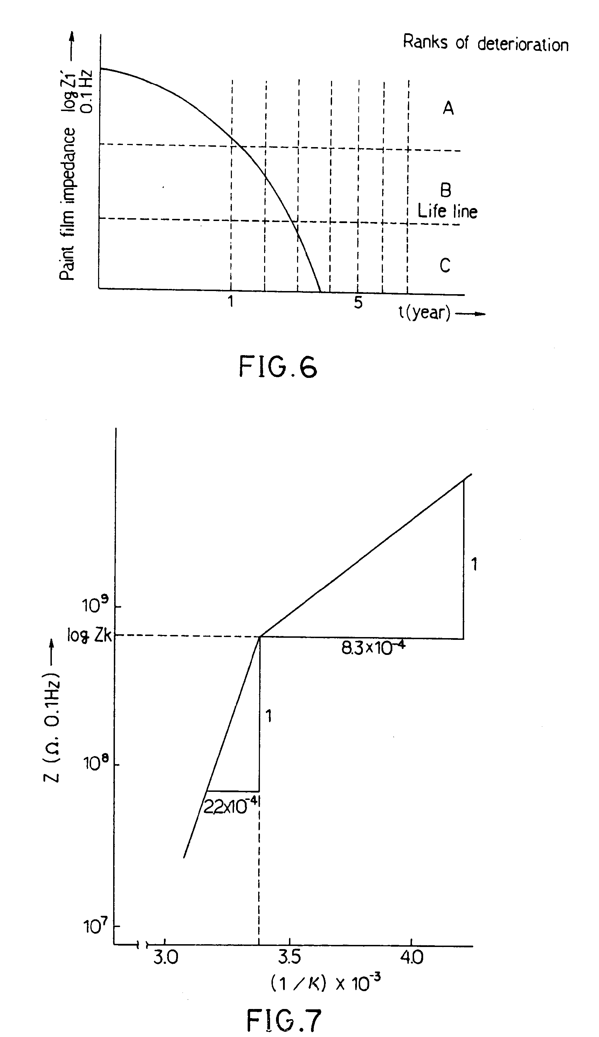

Now, the temperature compensation factor k is obtained as follows when the paint film 9 the paint material data of which is inputted at step S2 has the relationship shown in FIG. 7. That is, when the temperature is below 25°C, the value of log Z is obtained by the following equation:

$$\log Z = \log Zk + \frac{1}{8.3 \times 10^{-4}} \left( \frac{1}{T} - \frac{1}{298} \right)$$

where Zk= impedance at 25°C (298°K)

Similarly, when the temperature is 25°C or above, the value of log Z is obtained by the following equation:

$$\log Z = \log Zk + \frac{1}{2.2 \times 10^{-4}} \left( \frac{1}{T} - \frac{1}{298} \right)$$

Since the temperature compensation factor k is represented as k=Zk/Z, the factor k is obtained from the above-described equation when the temperature T is below 298°K (25°C):

$$k = \exp \left\{ \frac{\log_e 10}{8.3 \times 10^{-4}} \left( \frac{1}{298} - \frac{1}{T} \right) \right\}$$

In the same way, the factor k is obtained as follows when the temperature T is 298°K (25°C) or above:

$$k = \exp\left\{\frac{\log_e 10}{2.2 \times 10^{-4}}\left(\frac{1}{298} - \frac{1}{T}\right)\right\}$$

Step 7: drawing a diagram of an assumed life line:

As described above, the logarithm log Z of the paint film impedance Z has a linearity relative to the time t. An initial impedance $Z_0$ under the condition that the reference temperature is below 25°C with respect to the kind of paint whose data has been inputted at step S2 is stored as known data. The data of the number of elapsing years after formation of the paint film has also been inputted at step S2. Based on these data, the assumed life line diagram can be drawn by linking by the straight line L the logarithm log $Z_0$ of the initial impedance $Z_0$ to logarithm log $Z_1'$ of the paint film impedance converted to the detected paint film impedance $Z_1$ at the reference temperature. The life expectancy tr in FIG. 2 refers to a period of time represented by a section from the time of measurement to the point at which a line extended from the straight line L. The life line R can be previously determined based on degree of the paint film deterioration, degree of rusting on the base metal and the paint film impedance after an exposure test or accelerating test is carried out for the paint film. In the assumed life line graph, an axis of abscissa may be represented by t squared in consideration of the assuming accuracy, as shown in FIG. 6.

The above-described straight line L is obtained by a microcomputer having a program of a functional expression corresponding to an orthogonal graph by finding an equation of a line passing through the initial impedance $Z_0$ and a measured impedance $Z_1$. When a predetermined life-end-point impedance of the paint film is substituted into the equation, the point of intersection between two lines R and L is obtained.

Step S8: prediction of degree of deterioration and the time of repainting:

The degree of deterioration is ranked depending upon what range the value of the paint film impedance $Z_1'$ compensated for with respect to the temperature belongs to. In the embodiment, the paint film deterioration is classified into three ranks A, B and C and information such as shown in FIG. 4 is on the display 6, for example. When the paint film is determined to be at the rank B, the number of years until repainting (remaining life tr) obtained from the above-described assume life line is displayed.

Step S9: presuming a main cause of the deterioration:

Subsequently, when the personal computer 1 is operated so that a presuming program starts, an expert inference is carried out in an interactive mode so that a main cause of paint film deterioration is inferred based on the result of the measured paint film impedance $Z_1'$. In this program, information about condition of environment of the measured equipment is inputted to the personal computer 1. The result of the inference is displayed on the display 6. The analyzing program is completed after completion of the expert inference or when the deterioration cause presuming program is not started. The result of the deterioration diagnosis and the graph in the midst of analysis can be printed out.

CPU 2 operated in accordance with the above-described diagnosis program thus serves as determination means for performing an analyzing job based on the data of the paint film impedance Z and determining degree of deterioration of the paint film. The result of determination is displayed on the display 6. Consequently, a proper determination can be made about the degree of the paint film deterioration no matter who may operate the diagnosis device. Furthermore, the determination of the paint film deterioration can be performed with ease and accuracy.

The degree of deterioration of the paint film is determined based on the paint film impedance $Z_1'$ obtained by reducing the detected paint film impedance $Z_1$ to the value corresponding to the temperature value below the reference temperature. Consequently, even when the impedance of the paint film is varied by the temperature thereof, an exact determination of the degree of the paint film deterioration can be made.

Although the obtained determination is displayed on the display 6 in the foregoing embodiment, it may be printed out by a printer connected to the personal computer 1. Although the personal computer 1 employed in the foregoing embodiment is a general purpose computer, a dedicated purpose personal computer incorporating a display and a printer may be employed. The I/F board 8 attached to the personal computer 1 may or may not be provided with the D/A and A/D functions. Furthermore, circuitry 14 for the A/D conversion and D/A conversion may be externally provided, as is shown as a modified form in FIG. 9.

In the foregoing embodiment, the temperature of the paint film is sensed together with the measurement of the paint film impedance so that the sensed temperature is employed as an element for determination of the degree of the paint film deterioration. Such a temperature sensing means may be provided if necessary.

The foregoing disclosure and drawings are merely illustrative of the principles of the present invention and are not to be interpreted in a limiting sense. The only limitation is to be determined from the scope of the appended claims.

## Claims

1. A method of diagnosing the degree of deterioration of a paint film (9) based on an impedance comprising the steps of measuring an impedance by applying a probe (10) to the surface of the paint film (9), applying voltage across the probe (10) and a base metal (11) on which the paint film (9) is formed, the voltage having a frequency range of 0.01 to 1 Hz, and measuring the resulting current based upon the applied voltage across the probe (10), characterized by the steps of:

   a) converting an analog signal indicative of the measured current to a corresponding digital impedance signal;

   b) providing a microcomputer (2) with an operational program representative of a functional expression corresponding to an orthogonal graph having an axis representative of logarithmic values of impedance and an axis representative of an elapsed service time period in the year, the operational program including means (2) for converting an impedance value to a logarithmic value;

   c) inputting to the microcomputer (2) digital data of an initial impedance of the paint film (9) measured at the time of formation of the paint film (9), the digital impedance signal, digital data of an elapsed time period from the time of formation of the paint film (9) until the measurement of impedance of the paint film (9), and a predetermined life-end-point impedance of the paint film (9);

   d) performing an operation for obtaining an equation representative of a straight line passing through the initial impedance and the measured impedance on said orthogonal graph;

   e) performing an operation for obtaining in the microcomputer a life-end-point of the paint film (9) by substituting the predetermined life-end-point impedance into the equation representative of the straight line passing through the initial impedance and the measured impedance;

   f) obtaining, in the microcomputer (2), a remaining life-duration of the paint film (9) from the time of measurement of the impedance of the paint film (9) to the life end point; and

   g) displaying the obtained remaining life-duration of the paint film (9) on a display (6).

2. A method according to claim 1, characterized in that the voltage applied across the probe (10) and the base metal (11) has a plurality of difference frequencies.

3. A method according to claim 1, characterized in that the measured impedance of the paint film (9) is compensated for according to the temperature of the paint film (9) at the time of measurement of the impedance thereof by using the following equation and employing the compensated value ($Z_k$) for the measured impedance:

$$\log\ Z_k = \log\ Z - \frac{1}{\alpha}\left(\frac{1}{T} - \frac{1}{298}\right)$$

where Z is the measured impedance, $\alpha$ is a temperature compensation factor, and T is an absolute temperature (°K).

4. An apparatus for diagnosing the degree of deterioration of a paint film (9) based on an impedance comprising means for measuring an impedance by applying a probe (10) to the surface of the paint film (9), applying voltage across the probe (10) and a base metal (11) on which the paint film (9) is formed, the voltage having a frequency range 0.01 to 1 Hz, and measuring the resulting current based upon the applied voltage across the probe (10), characterized by:

   a) means (8) for converting an analog signal indicative of the measured current to a corresponding digital impedance signal;

   b) means (5a) for providing a microcomputer (2) with an operational program representative of a functional expression corresponding to an orthogonal graph having an axis representative of logarithmic values of impedance and an axis representative of an elapsed service time period in the year, the operational program including means (2) for converting an impedance value to a logarithmic value;

c) means (8) for inputting to the microcomputer (2) digital data of an intial impedance of the paint film (9) measured at the time of formation of the paint film (9), the digital impedance signal, digital data of an elapsed time period from the time of formation of the paint film (9) until the measurement of the impedance of the paint film (9), and a predetermined life-end-point impedance of the paint film (9);

d) means (2) for performing an operation for obtaining an equation representative of a straight line passing through the initial impedance and the measured impedance of said orthogonal graph;

e) means (2) for performing an operation for obtaining in the microcomputer a life-end-point of the paint film (9) by substituting the predetermined life-end-point impedance into the equation representative of the straight line passing through the initial impedance and the measured impedance;

f) means (2) for obtaining, in the microcomputer (2), a remaining life-duration of the paint film (9) from the time of measurement of the impedance of the paint film (9) to the life end point; and

g) means (2) for displaying the obtained remaining life-duration of the paint film (9) on a display (6).

## Patentansprüche

1. Verfahren zur Diagnose des Grades der Qualitätsminderung einer Farbschicht (9) auf der Grundlage einer Impedanz mit den folgenden Schritten: Messen einer Impedanz durch Anlegen einer Sonde (10) an die Oberfläche der Farbschicht (9), Anlegen einer Spannung zwischen die Sonde (10) und das Trägermetall (11), auf dem die Farbschicht (9) ausgebildet ist, wobei die Spannung einen Frequenzbereich von 0.01 bis 1 Hz hat, und Messen des resultierenden Stroms auf der Grundlage der an die Sonde (10) angelegten Spannung, gekennzeichnet durch die folgenden Schritte:

   a) Umwandeln eines Analogsignals, das den gemessenen Strom anzeigt, in ein entsprechendes digitales Impedanzsignal;

   b) Versorgen eines Mikrocomputers (2) mit einem Betriebsprogramm, das einen Funktionsausdruck darstellt, der einem orthogonalen Diagramm mit einer Achse, die logarithmischen Werten der Impedanz darstellt, und einer Achse, die eine vergangene Beanspruchungszeitdauer im Jahr darstellt, entspricht, wobei das Betriebsprogramm Mittel (2) zum Umwandeln eines Impedanzwertes in einen logarithmischen Wert aufweist:

   c) Eingeben, in den Mikrocomputer (2), von Digitaldaten einer Anfangsimpedanz der Farbschicht (9), die zu Zeit der Ausbildung der Farbschicht (9) gemessen wird, des digitalen Impedanzsignals, von Digitaldaten einer vergangenen Zeitdauer von der Zeit der Ausbildung der Farbschicht (9) bis zur Messung der Impedanz der Farbschicht (9) und einer vorbestimmten Lebensdauerendpunktimpedanz der Farbschicht (9);

   d) Durchführen eines Vorgangs zum Erlangen einer Gleichung, die eine gerade Linie darstellt, die durch die Anfangsimpedanz und die gemessene Impedanz in dem orthogonalen Diagramm läuft;

   e) Durchführung eines Vorgangs zum Erlangen, im Mikrocomputer, eines Lebensdauerendpunkts der Farbschicht (9), indem die vorbestimmte Lebensdauerendpunktimpedanz in die Gleichung eingesetzt wird, die die gerade Linie darstellt, die durch die Anfangsimpedanz und die gemessene Impedanz läuft;

   f) Erlangen, im Mikrocomputer (2), einer verbleibenden Lebensdauer der Farbschicht (9) von der Zeit der Messung der Impedanz der Farbschicht (9) bis zum Lebensdauerendpunkt; und

   g) Anzeigen der erlangten verbleibenden Lebensdauer der Farbschicht (9) auf der Anzeige (6).

2. Verfahren nach Anspruch 1, dadurch gekennzeichnet, daß die Spannung, die zwischen die Sonde (10) und das Trägermetall (11) angelegt wird, mehrere Differenzfrequenzen hat.

3. Verfahren nach Anspruch 1, dadurch gekennzeichnet, daß die gemessene Impedanz der Farbschicht (9) entsprechend der Temperatur der Farbschicht (9) zur Zeit der Messung ihrer Impedanz kompensiert wird, indem die folgende Gleichung verwendet wird und der kompensierte Wert $Z_k$ für die gemessene Impedanz verwendet wird;

$$\log Z_k = \log Z - \frac{1}{\alpha}\left(\frac{1}{T} - \frac{1}{298}\right)$$

wobei Z die gemessene Impedanz ist, $\alpha$ ein Temperaturkompensationsfaktor und T eine absolute Temperatur (°K) ist.

4. Vorrichtung zur Diagnose des Grades der Qualitätsminderung einer Farbschicht (9) auf der Grundlage einer Impedanz mit Mitteln zum Messen einer Impedanz durch Anlegen einer Sonde (10) an die Oberfläche der Farbschicht (9), Anlegen einer Spannung zwischen die Sonde (10) und ein Trägermetall (11), auf dem die Farbschicht (9) ausgebildet ist, wobei die Spannung einen Frequenzbereich von 0.01 bis 1 Hz hat, und Messen des resultierenden Stroms auf der Grundlage der an die Sonde (10) angelegten Spannung, gekennzeichnet durch:

a) Mittel (8) zum Umwandeln eines Analogsignals, das den gemessenen Strom anzeigt, in ein entsprechendes digitales Impedanzsignal;

b) Mittel (5a) zum Versorgen eines Mikrocomputers (2) mit einem Betriebsprogramm, das einen Funktionsausdruck darstellt, der einem orthogonalen Diagramm mit einer Achse, die logarithmische Werte der Impedanz darstellt, und einer Achse, die eine vergangene Beanspruchungszeitdauer im Jahr darstellt, entspricht, wobei das Betriebsprogramm Mittel (2) zum Umwandeln eines Impedanzwertes in einen logarithmischen Wert aufweist;

c) Mittel (8) zum Eingeben, in den Mikrocomputer (2), von Digitaldaten einer Anfangsimpedanz der Farbschicht (9), die zur Zeit der Ausbildung der Farbschicht (9) gemessen wird, des digitalen Impedanzsignals, von Digitaldaten einer vergangenen Zeitdauer von der Zeit der Ausbildung der Farbschicht (9) bis zur Messung der Impedanz der Farbschicht (9) und einer vorbestimmten Lebensdauerendpunktimpedanz der Farbschicht (9);

d) Mittel (2) zum Durchführen eines Vorgangs zum Erlangen einer Gleichung, die eine gerade Linie darstellt, die durch die Anfangsimpedanz und die gemessene Impedanz des orthogonalen Diagramms läuft;

e) Mittel (2) zum Durchführen eines Vorgangs zum Erlangen, im Mikrocomputer, eines Lebensdauerendpunkts der Farbschicht (9), indem die vorbestimmte Lebensdauerendpunktimpedanz in die Gleichung eingesetzt wird, die die gerade Linie darstellt, die durch die Anfangsimpedanz und die gemessene Impedanz läuft;

f) Mittel (2) zum Erlangen, im Mikrocomputer (2), einer verbleibenden Lebensdauer der Farbschicht (9) von der Zeit der Messung der Impedanz der Farbschicht (9) bis zum Lebensdauerendpunkt; und

g) Mittel (2) zum Anzeigen der erlangten verbleibenden Lebensdauer der Farbschicht (9) auf einer Anzeige (6).

## Revendications

1. Procédé de diagnostic du degré de détérioration d'un film de peinture (9) basé sur une impédance, comprenant les étapes de mesure d'une impédance en appliquant une sonde (10) sur la surface du film de peinture (9), d'application d'une tension entre la sonde (10) et un métal de base (11) sur lequel le film de peinture (9) est formé, la tension présentant une plage de fréquences de 0.01 à 1 Hz, et de mesure du courant résultant sur la base de la tension appliquée à la sonde (10), caractérisé par les étapes de:

a) conversion d'un signal analogique indicatif du courant mesuré en un signal d'impédance numérique correspondant;

b) fourniture d'un micro-ordinateur (2) muni d'un programme opérationnel représentatif d'une expression fonctionnelle correspondant à un graphique orthogonal comportant un axe représentatif de valeurs logarithmiques d'impédance et un axe représentatif d'une période temporelle de service écoulée dans l'année, le programme opérationnel incluant un moyen (2) pour convertir une valeur d'impédance en une valeur logarithmique;

c) entrée sur le micro-ordinateur (2) de données numériques d'une impédance initiale du film de peinture (9) mesurée à l'instant de la formation du film de peinture (9), du signal d'impédance numérique, des données numériques d'une période temporelle écoulée depuis l'instant de la formation du film de peinture (9) jusqu'à la mesure d'impédance du film de peinture (9), et d'une impédance de point de fin de vie prédéterminée du film de peinture (9);

d) réalisation d'une opération pour obtenir une équation représentative d'une ligne rectiligne passant par l'impédance initiale et l'impédance mesurée sur ledit graphique orthogonal;

e) réalisation d'une opération pour obtenir dans le micro-ordinateur un point de fin de vie du film de peinture (9) en substituant l'impédance de point de fin de vie prédéterminée dans l'équation représentative de la ligne rectiligne qui passe par l'impédance initiale et par l'impédance mesurée;

f) obtention dans le micro-ordinateur (2) d'une durée de vie restante du film de peinture (9) depuis l'instant de la mesure de l'impédance du film de peinture (9) jusqu'au point de fin de vie; et

g) affichage de la durée de vie restante obtenue du film de peinture (9) sur un affichage (6).

2. Procédé selon la revendication 1, caractérisé en ce que la tension appliquée entre la sonde (10) et le métal de base (11) comporte une pluralité de fréquences de différence.

3. Procédé selon la revendication 1, caractérisé en ce que l'impédance mesurée du film de peinture (9) est compensée quant à la température du film de peinture (9) à l'instant de la mesure de son impédance en utilisant l'équation suivante et en utilisant la valeur compensée ($Z_k$) quant à l'impédance mesurée

$$\log Z_k = \log Z - \frac{1}{\alpha}\left(\frac{1}{T} - \frac{1}{298}\right)$$

où Z est l'impédance mesurée, $\alpha$ est un facteur de compensation de température et T est une température absolue (°K).

4. Appareil pour diagnostiquer le degré de détérioration d'un film de peinture (9) sur la base d'une impédance, comprenant un moyen pour mesurer une impédance en appliquant une sonde (10) sur la surface du film de peinture (9), pour appliquer une tension entre la sonde (10) et un métal de base (11) sur lequel le film de peinture (9) est formé, la tension présentant une plage de fréquences de 0.01 à 1 Hz, et pour mesurer le courant résultant sur la base de la tension appliquée à la sonde (10), caractérisé par:

a) un moyen (8) pour convertir un signal analogique indicatif du courant mesuré en un signal d'impédance numérique correspondant;

b) un moyen (5a) pour munir un micro-ordinateur (2) d'un programme opérationnel représentatif d'une expression fonctionnelle correspondant à un graphique orthogonal comportant un axe représentatif de valeurs logarithmiques d'impédance et un axe représentatif d'une période temporelle de service écoulée dans l'année, le programme opérationnel incluant un moyen (2) pour convertir une valeur d'impédance en une valeur logarithmique;

c) un moyen (8) pour entrer sur le micro-ordinateur (2) des données numériques d'une impédance initiale du film de peinture (9) mesurée à l'instant de la formation du film de peinture (9), le signal d'impédance numérique, des données numériques d'une période temporelle écoulée depuis l'instant de la formation du film de peinture (9) jusqu'à la mesure de l'impédance du film de peinture (9) et une impédance de point de fin de vie prédéterminée du film de peinture (9);

d) un moyen (2) pour réaliser une opération pour obtenir une équation représentative d'une ligne rectiligne qui passe par l'impédance initiale et par l'impédance mesurée dudit graphique orthogonal;

e) un moyen (2) pour réaliser une opération pour obtenir dans le micro-ordinateur un point de fin de vie du film de peinture (9) en substituant l'impédance de point de fin de vie prédéterminée dans l'équation représentative de la ligne rectiligne qui passe par l'impédance initiale et par l'impédance mesurée;

f) un moyen (2) pour obtenir, dans le micro-ordinateur (2), une durée de vie restante du film de peinture (9) depuis l'instant de la mesure de l'impédance du film de peinture (9) jusqu'au point de fin de vie; et

(g) un moyen (2) pour afficher la durée de vie restante obtenue du film de peinture (9) sur un affichage (6).

FIG.1

FIG.2

FIG.3

FIG.4{

(1) Prediction of degree of deterioration ———————————

The paint film deterioration is classified into the following ranks A to C:

Rank A:   The paint film is in a sound condition though reduced gloss and choking are perceived to some extent.

Rank B:   Corrosion of the base metal is in progress though rust cannot be visually perceived.

Rank C:   Rust is conspicuous and rust prevention effect of the paint film has been reduced.

"Deterioration of the paint film is presumed to be in **rank B** as the result of the present measurement."

(2) Prediction of time of repainting ———————————

The time of repainting refers to a stage in which the base metal has begun to develop symptoms of rust. When the repainting is carried out at this stage, a most economic corrosion preventive paint can be obtained without a serious rusting on the base metal under the paint film.

The time of repainting can be predicted as follows as the result of the present measurement:

"It would be better to consider repainting within **0.4** years since a symptom of deterioration can be perceived."

**FIG.5**

FIG.6

FIG.7

15

FIG.8

FIG.9

16